(19) Europäisches Patentamt
European Patent Office
Office européen des brevets

(11) Veröffentlichungsnummer: **0 008 123**
**A1**

(12) **EUROPÄISCHE PATENTANMELDUNG**

(21) Anmeldenummer: **79102893.9**

(22) Anmeldetag: **10.08.79**

(51) Int. Cl.³: **C 07 D 413/10,** C 07 D 417/10, D 06 L 3/12, C 08 K 5/35, C 08 K 5/47, C 08 J 3/20

(30) Priorität: **14.08.78 DE 2835540**

(43) Veröffentlichungstag der Anmeldung: **20.02.80 Patentblatt 80/4**

(84) Benannte Vertragsstaaten: **AT BE CH DE FR GB IT NL SE**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Erckel, Rüdiger, Dr., Staufenstrasse 16, D-6239 Eppstein/Taunus (DE)**
Erfinder: **Eckes, Helmut, Dr., Feldbergblick 5, D-6239 Eppstein/Taunus (DE)**
Erfinder: **Rösch, Günter, Hohlweg 17, D-6232 Bad Soden am Taunus (DE)**

(54) **Neue Diamino-1,3,5-triazinylstilben-Verbindungen, Verfahren zu ihrer Herstellung und ihre Verwendung als optische Aufheller.**

(57) Diamino-1,3,5-triazinylstilben-Verbindungen der allgemeinen Formel

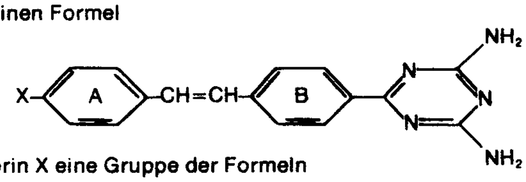

(1)

worin X eine Gruppe der Formeln

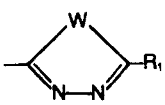

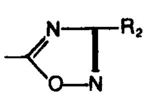

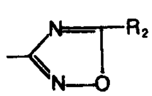

bedeutet und $R_1$ eine eventuell substituierte Aryl- oder Aralkenylgruppe oder eine heteroaromatische Gruppe ist, $R_2$ die gleiche Bedeutung wie $R_1$ hat und zusätzlich eine $(C_1–C_{18})$-Alkyl-, Cycloalkyl-, Aralkyl-, Alkoxyalkyl-, Halogenalkyl-, Hydroxyalkyl-, Alkylaminoalkyl-, Carboxyalkylgruppe oder eine Gruppe der Formel $-(CH_2CH_2O)_n$-R' mit R' = Wasserstoff oder $(C_1–C_4)$-Alkyl und n = 1 oder 2 bedeuten, W ein Sauerstoff oder Schwefelatom bedeutet und die Ringe A und B noch weitere nichtchromophore Substituenten aufweisen können. Verfahren zu ihrer Herstellung und ihre Verwendung als optische Aufheller.

EP 0 008 123 A1

HOECHST AKTIENGESELLSCHAFT HOE 78/F 169 Dr.OT/St

**Neue Diamino-1,3,5-triazinylstilben-Verbindungen, Verfahren zu ihrer Herstellung und ihre Verwendung als optische Aufheller**

Die vorliegende Erfindung betrifft neue Diamino-1,3,5-triazinylstilbenverbindungen, Verfahren zu deren Herstellung sowie deren Verwendung als optische Aufhellmittel für organische Materialien. Die neuen Verbindungen entsprechen der Formel

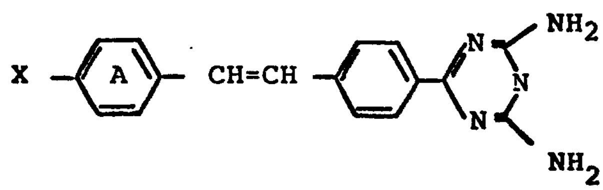

(I)

worin X eine Gruppe der Formeln

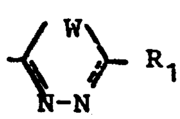

(Ia)

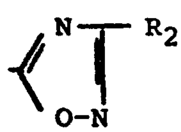

(Ib) oder

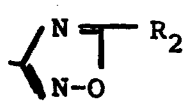

(Ic)

bedeutet und $R_1$ eine eventuell substituierte Aryl- oder Aralkenylgruppe oder eine heteroaromatische Gruppe ist, $R_2$ die gleiche Bedeutung wie $R_1$ hat und zusätzlich eine $(C_1-C_{18})$-Alkyl-, Cycloalkyl-, Aralkyl-, Alkoxyalkyl-, Halogenalkyl-, Hydroxyalkyl-, Alkylaminoalkyl-, Carboxyalkylgruppe oder eine Gruppe der Formel $-(CH_2CH_2O)_n-R'$ mit R' = Wasserstoff oder $(C_1-C_4)$-Alkyl und n = 1 oder 2 bedeuten, W ein Sauerstoff oder Schwefelatom bedeutet und die Ringe A und B noch weitere nicht-chromophore Substituenten aufweisen können.

Bevorzugt sind solche Verbindungen der allgemeinen Formel I, worin W ein Sauerstoffatom, $R_1$ einen Phenylring der durch

ein oder zwei Chloratome, eine oder zwei $(C_1-C_4)$-Alkyl- oder $(C_1-C_4)$Alkoxy-$(C_1-C_4)$-alkyl-gruppen, eine Phenyl-, Cyano-, Carbonsäure-, Carbo-$(C_1-C_4)$-alkoxy-, Carbonsäureamid-, Sulfonsäure-, Sulfonsäureamid- oder Sulfonsäure-$(C_1-C_4)$-alkylestergruppe substituiert sein kann, $R_2$ die gleiche Bedeutung wie $R_1$ hat und zusätzlich jeweils eine $(C_1-C_6)$-Alkyl-, $(C_1-C_6)$-Chloralkyl-, $(C_1-C_6)$-Alkoxy-$(C_1-C_4)$-alkyl-, $(C_1-C_4)$-Hydroxyalkyl-, Benzylgruppe oder eine Grupp der Formel $-(CH_2CH_2O)_n-R'$ mit n = 1 oder 2 R' = Wasserstoff oder $(C_1-C_4)$-Alkyl bedeutet.

Insbesondere bevorzugt sind solche Verbindungen der allgemeinen Formel I, wobei $R_1$ Phenyl, mono- oder di-$(C_1-C_2)$-Alkylphenyl, $(C_1-C_2)$-Alkoxyphenyl, Chlorphenyl oder Dichlorphenyl, $R_2$ die gleiche Bedeutung wie $R_1$ hat und zusätzlich jeweils $(C_1-C_5)$-Alkyl, $(C_1-C_2)$-Chloralkyl, $(C_1-C_2)$-Hydroxyalkyl, $(C_1-C_2)$-Alkoxyalkyl oder Benzyl, W Sauerstoff bedeutet und die Ringe A und B unsubstituiert sind.

Ganz besonders bevorzugt sind Verbindungen der Formel

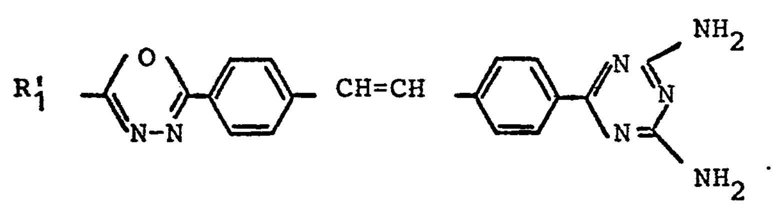 2

worin $R_1'$ Phenyl, mono oder di-$(C_1-C_2)$-Alkylphenyl, Chlorphenyl oder Dichlorphenyl bedeutet.

Unter nicht-chromophoren Substituenten der Ringe A und B werden z.B. verstanden Alkyl mit 1 bis 12 Kohlenstoffatomen, Cyclohexyl, Phenylalkyl mit 1 bis 3 Kohlenstoffatomen im Alkylteil; unsubstituiertes oder mit 1 oder 2 Substituenten aus der Reihe Chlor, Methyl oder Methoxy substituiertes Phenyl; Alkoxy mit 1 bis 4 Kohlenstoffatomen; unsubstituiertes oder mit 1 oder 2 Substituenten aus der Reihe Chlor, Methyl oder Methoxy substituiertes Phenoxy; Chlor, Fluor, Brom, Cyano, -COOY, worin Yfür Wasserstoff, ein salzbildendes Kation, Alkyl mit 1 bis 5 Kohlenstoffatomen oder Benzyl steht; CONY' ($Y_1'$), worin Y' für Wasserstoff, Alkyl mit 1 bis 6 Kohlenstoffatomen, Hydroxyalkyl mit 1 bis 4 Kohlenstoffatomen, Alkoxyalkyl mit 2 bis 8 Kohlenstoffatomen, Phenyl oder Benzyl und $Y_1'$ für Wasserstoff, Alkyl mit 1 bis 6 Kohlenstoffatomen, Hydroxyalkyl mit 1 bis 4 Kohlenstoffatomen oder Alkoxyalkyl mit 2 bis 8 Kohlenstoffatomen oder Y' und $Y_1'$ zusammen mit dem Stickstoff für einen Morpholino- oder Piperidinorest stehen; oder abgewandelte Sulfogruppen wie $-SO_2OY$, worin Y die vorstehend angegebene Bedeutung hat, $-SO_2NY'$ ($Y_1'$), worin Y' und $Y_1'$ die vorstehend angegebene Bedeutung haben; Alkylsulfonyl mit 1 bis 6 Kohlenstoffatomen; Benzylsulfonyl oder unstubstituiertes oder mit Chlor oder Methyl substituiertes Phenylsulfonyl; oder im Falle von zwei ortho-ständigen Substituenten auch Alkylen mit 3 oder 4 Kohlenstoffatomen oder 1,3-Butadienylen.

Die Diamino-1,3,5-triazinyl-stilbenverbindungen der Formel I werden in an sich bekannter Weise gemäß Org.Synth. Coll. Vol IV, 78 aus der Carbonsäure

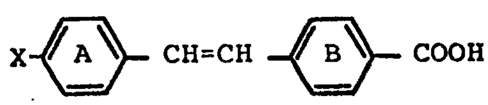

3

oder deren Derivaten hergestellt, worin X, A und B die oben angegebene Bedeutung haben, beispielsweise aus dem Nitril durch Umsetzung mit Dicyandiamid in An- oder Abwesenheit basischer Katalysatoren oder aus dem entsprechenden Amidinsalz der Säure der Formel 3 durch Umsetzung mit Dicyandiamid oder Biguanidsalzen oder durch Umsetzung des entsprechenden Chlorids der Säure der Formel 3 mit Biguanidsalzen oder durch Umsetzung des entsprechenden Nitrils der Säure der Formel 3 mit Harnstoff und Ammoniak.

Bevorzugte Darstellungsweise ist die Umsetzung des Nitrils der Carbonsäure (3) mit Dicyandiamid unter Zusatz basischer Katalysatoren in Gegenwart polarer Lösungsmittel bei Temperaturen von 70 - 250°C, gegebenenfalls unter Druck. Geeignete Lösungsmittel sind beispielsweise Methanol, Ethanol, i-Propanol, tert. Butanol, Cyclohexanol, Methylglykol, Methyldiglykol, Butylglykol, Diglykol, Dimethylformamid, Phosphorsäuretrisdimethylamid, Dimethylsulfoxid, Methylpyrrolidon.

Geeignete basische Katalysatoren sind Alkalicarbonate, -hydroxide, -amide und alkoholate sowie primäre, sekundäure und tertiäre Amine oder quartäre Ammoniumbasen. Die Alkoholate können auch durch Auflösen von Alkalimetall, -amid oder -hydrid in dem als Lösungsmittel verwendeten Alkohol hergestellt werden.

Die als Ausgangsverbindung dienende Carbonsäure 3 bzw. deren funktionell abgewandelte Derivate sind literaturbekannt bzw. lassen sich nach bekannten Verfahren herstellen. Die freie Carbonsäure 3 läßt sich nach ebenfalls bekannten Verfahren über das Säurechlorid in das Amid überführen, das dann mit einem wasserabspaltenden Mittel zum Nitril umgesetzt wird. Auf diesem Weg lassen sich die freie Säure 3 und deren funktionelle Derivate ineinander umwandeln.

Für die Herstellung von Verbindungen der Formel 3 seien beispielsweise folgende beiden Wege genannt:

a) Umsetzung von Verbindungen der Formel

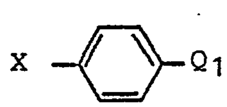 (4)

mit Verbindungen der Formel

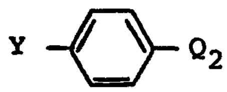 (5)

wobei X die oben angegebene Bedeutung besitzt, Y für eine Carbonamido-, Cyan-, Carbalkoxy- oder Carbonsäuregruppe steht und wobei eines der Symbole $Q_1$ oder $Q_2$ eine Aldehydgruppe und das andere Symbol eine Gruppe der Formel

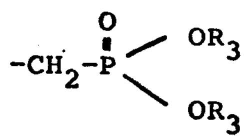 (6a)

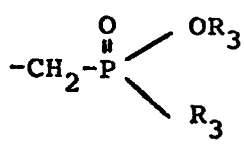 (6b)

oder 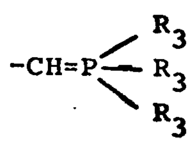 (6c)

bedeutet, wobei $R_3$ einen gegebenenfalls substituierten Alkylrest, mit vorzugsweise 1 - 6 C-Atomen, einen Arylrest, vorzugsweise Phenyl, einen Cycloalkylrest oder einen Aralkylrest, vorzugsweise Benzyl darstellt.

b) Umsetzung von Verbindungen der Formel

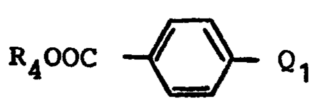 (7)

wobei $R_4$ für Wasserstoff oder $C_1$-$C_4$ Alkyl steht mit Verbindungen der Formel

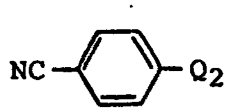 (8)

wobei $Q_1$ und $Q_2$ die oben angegebene Bedeutung besitzen, zu Verbindungen der Formel

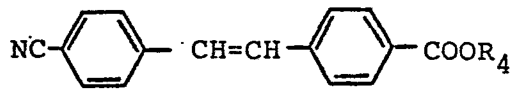

(9a)

Falls $R_4$ Wasserstoff bedeutet, erhält man aus dieser Verbindung direkt durch Chlorierung das Säurechlorid der Formel

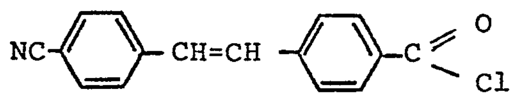

(9b)

Wenn $R_4$ eine Alkylgruppe darstellt, muß der Ester zuvor zur freien Säure verseift werden.

Die Umsetzung von Verbindungen der Formel 9b mit Verbindungen der Formel

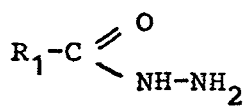

(10)

und nachfolgende Cyclisierung mit einem wasserabspaltenden Mittel wie Phosphorpentoxid führt zu den der Ausgangsverbindung 3 entsprechenden Nitrilen mit der Formel

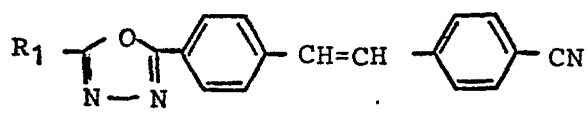

(11)

Diese Verbindung (11) läßt sich auch in der Weise herstellen, daß man das Säurechlorid (9b) zunächst mit Hydrazidhydrat zum Hydrazid umsetzt, dieses Hydrazid mit dem Säurechlorid der Formel

$R_1$-COCl

reagieren läßt und anschließend wieder mit einem wasserabspaltenden Mittel den Ring schließt. Falls W in der Formel 1a ein Schwefelatom darstellt, erfolgt die Cyclisierung mit $P_2S_5$.

Wird die Verbindung der Formel (9b) mit Verbindungen der Formel

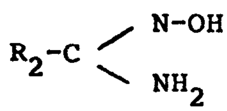

(12)

umgesetzt, so erhält man die der Ausgangsverbindung 3 entsprechenden Nitrile der Formel

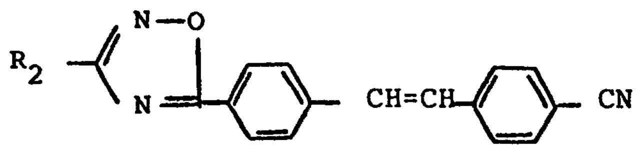

(13)

Die Nitrile der Formel 11 und 13 können dann, wie bereits oben geschildert, mit Dicyandiamid zu den Verbindungen 1a und 1b umgesetzt werden.

Anstelle der Verbindung (9b) kann man auch von einer Verbindung der Formel

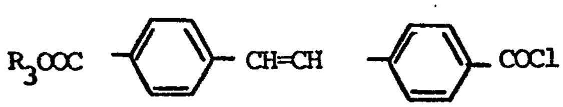

ausgehen. Man erhält dann Verbindungen analog zu den Verbindungen der Formeln 11 bzw. 13. Die Carboalkoxygruppe kann dann nach bekannten Verfahren über die freie Säure, Säurechlorid und Säureamid in das Nitril umgewandelt werden, so daß man auch auf diesem Wege die Verbindungen 11 bzw. 13 erhält.

Verbindungen der Formel Ic sind beispielsweise auf folgendem Weg zugänglich.

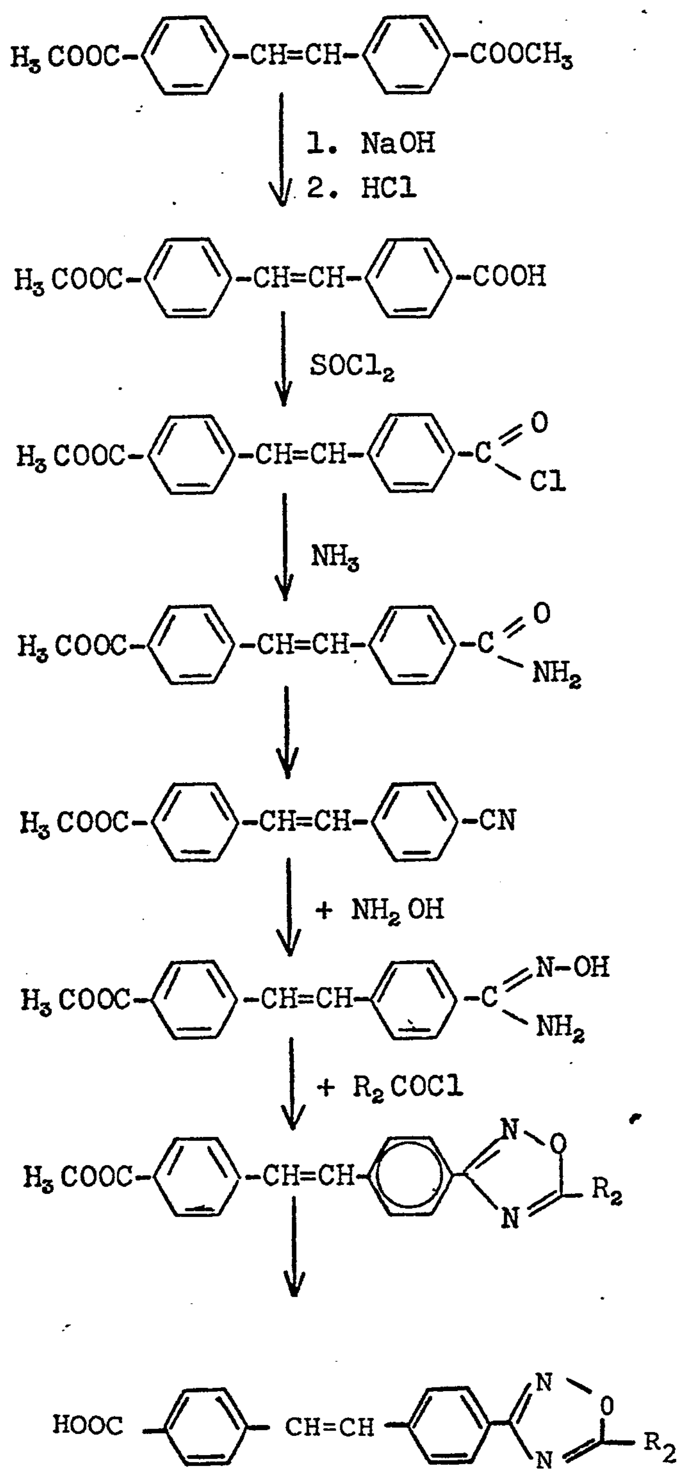


(14)

(15)

(16)

(17)

(18)

(19a)

(19b)

(20)

Diese Verbindung entspricht der Ausgangsverbindung 3

Da die Verbindung 18 unter die Formel 9a fällt, ist diese Verbindung auch auf dem unter a) geschilderten Weg erhältlich.

Die Verbindungen der Formel 4, 5, 7 und 8 sind bekannt bzw. lassen sich nach literaturbekannten Verfahren herstellen. Die Umsetzung von Verbindungen der Formeln 4 und 5 bzw. 7 und 8 erfolgt vorteilhaft in indifferenten Lösungsmitteln. Als Beispiele hierfür seien Kohlenwasserstoffe wie Toluol und Xylol oder Alkohole wie Methanol, Ethanol, Isopropanol, Butanol, Glykole, Glykoläther wie 2-Methoxyäthanol, Hexanole, Cyclohexanol und Cyclooctanol, ferner Äther wie Diisopropyläther, Tetrahydrofuran und Dioxan sowie Dimethylsulfoxyd, Formamid und N-Methylpyrrolidon genannt. Besonders geeignet sind polare organische Lösungsmittel wie Dimethylformamid, N-Methylpyrrolidon und Dimethylsulfoxid. Auch in wäßriger Lösung lassen sich einige der Umsetzungen durchführen.

Die Temperatur, bei welcher die Umsetzung durchgeführt wird, kann in weiten Grenzen schwanken. Sie wird bestimmt durch die Beständigkeit des verwendeten Lösungsmittels gegenüber den Reaktionsteilnehmern, insbesondere gegenüber den stark basischen Alkaliverbindungen, durch die Reaktivität der Kondensationspartner und durch die Wirksamkeit der Kombination Lösungsmittel-Base als Kondensationsmittel.

In der Praxis kommen im allgemeinen Temperaturen zwischen etwa 10 und 100°C in Betracht, insbesondere wenn Dimethylformamid oder Dimethylsulfoxid als Lösungsmittel verwendet werden. Der Temperatur-Vorzugsbereich liegt bei 20 bis 60°C. Es können jedoch unter Umständen auch höhere Temperaturen angewandt werden, wenn dies aus Gründen der Zeitersparnis erwünscht ist, oder ein weniger aktives, dafür aber billigeres Kondensationsmittel eingesetzt werden soll. Grundsätzlich sind somit auch Reaktionstemperaturen im Intervall von 10 bis 180°C möglich.

Als stark basische Alkaliverbindungen kommen vor allem die Hydroxyde, Amide und Alkoholate (vorzugsweise solche primärer, 1 bis 4 Kohlenstoffatome enthaltender Alkohole) der Alkalimetalle in Betracht, wobei aus ökonomischen Gründen solche des Natriums und Kaliums von vorwiegendem Interesse sind. Es können jedoch grundsätzlich und in besonderen Fällen auch Alkalisulfide und -carbonate, Arylalkaliverbindungen wie z.B. Phenyllithium oder stark basische Amine (einschließlich Ammoniumbasen, z.B. Trialkylammoniumhydroxyde) mit Erfolg verwendet werden.

Die Verbindungen der Formel 10 sind literaturbekannt bzw. können nach bekannten Verfahren beispielsweise durch Umsetzung von Verbindungen der Formel

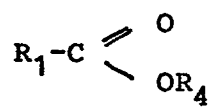

(21)

worin $R_1$ und $R_4$ die obenstehende Bedeutung besitzen, mit Hydrazinhydrat erhalten werden.

Die Amidoxime der Formeln 12 und 19a erhält man nach bekannten Verfahren durch Addition von Hydroxylamin an die entsprechenden Nitrile (DT-OS 2 709 924).

Die Umsetzung der Verbindungen der Formeln 9b mit 10 erfolgt nach bekannten Verfahren, beispielsweise in einem inerten organischen Lösungsmittel bei Temperaturen zwischen 0° - 200°C gegebenenfalls in Gegenwart von säurebildenden Mitteln und anschließenden Ringschluß des so erhaltenen Zwischenproduktes in Gegenwart von wasserabspaltenden Mitteln wie beispielsweise Thionylchlorid, Phosphoroxychlorid oder Phosphorpentachlorid, gegebenenfalls in einem inerten Lösungsmittel, bei Temperaturen von 20 - 200°C.

Die Umsetzung von Verbindungen der Formel 9b mit Verbindungen der Formel 12 oder von Verbindungen der Formel

19a mit Verbindung der Formel 19b erfolgt vorzugsweise in Gegenwart eines säurebindenden Mittels in einem inerten Lösungsmittel bei Temperaturen von 20 - 200°C.

Als Lösungsmittel für die Reaktion eignen sich z.B. Chlorbenzol, Di- oder Trichlorbenzol und insbesondere Dimethylformamid, N-Methylpyrrolidon oder Dimethylsulfoxyd. Als säurebindende Mittel sind beispielsweise Natriumcarbonat, Kaliumcarbonat, Calciumcarbonat, Triäthylamin oder Äthyldiisopropylamin verwendbar.

Die vorstehend definierten neuen Verbindungen zeigen in gelöstem oder feinverteiltem Zustand eine mehr oder weniger ausgeprägte Fluoreszenz. Sie eignen sich deshalb zum optischen Aufhellen der verschiedensten synthetischen, halbsynthetischen oder natürlichen organischen Materialien oder Substanzen, welche solche organischen Materialien enthalten.

Hierfür seien beispielsweise, ohne daß durch die nachfolgende Übersicht irgendeine Beschränkung hierauf ausgedrückt werden soll, die folgenden Gruppen von organischen Materialien, soweit eine optische Aufheller derselben in Betracht kommt, genannt:

I. Synthetische organische hochmolekulare Materialien:

a) Polymerisationsprodukte auf Basis mindestens eine polymerisierbare Kohlenstoff-Kohlenstoff-Doppelbindung enthaltender organischer Verbindungen, d.h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von α,ß-ungesättigten Carbonsäuren oder Derivaten solcher Carbonsäuren, insbesondere von Acrylverbindungen (wie z.B. Acrylestern, Acrylsäure, Acrylnitril, Acrylamiden und

deren Derivaten oder deren Methacryl-Analoga), von Olefin-Kohlenwasserstoffen (wie z.B. Äthylen, Propylen, Styrole oder Diene, ferner sogenannte ABS-Polymerisate), Polymerisate auf Basis von Vinyl- und Vinyliden-Verbindungen (wie z.B. Vinylchlorid, Vinylalkohol, Vinylidenchlorid),

b) Polymerisationsprodukte die durch Ringöffnung erhältlich sind z.B. Polyamide vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Äthylenglykolterephthalsäure-Polyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharz) Polyester, Polyamide (z.B. Hexamethylendiamin-adipat), Maleinatharze, Melaminharze, deren Vorkondensate und Analoga Polycarbonate, Silikone,

d) Polyadditionsprodukte wie Polyurethane (vernetzt und unvernetzt), Epoxidharze.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilden vorliegen, d.h. beispielsweise als vorwiegend dreidimensional ausgedehnte Körper wie Platten, Profile ., Spritzgußförmlinge, verschiedenartige Werkstücke, Schnitzel, Granulate oder Schaumstoffe, ferner als vorwiegend zweidimensional ausgebildete Körper wie Filme, Folien, Lacke, Überzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z.B. Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäß anzuwendenden Verbindungen kommt u.a. Bedeutung für die Behandlung von textilen organischen Materialien, insbesondere textilen Geweben, zu. Sofern Fasern, welche als Stapelfasern oder Endlosfäden, in Form von Strängen, Geweben, Gewirken, Vliesen, beflockten Substrate oder Verbundstoffe vorliegen können, erfindungsgemäß optisch aufzuhellen sind, geschieht dies mit Vorteil in wäßrigem Medium, worin die betreffenden Verbindungen in feinverteilter Form (Suspension, sogenannten Mikrodispersionen, gegebenenfalls Lösungen), vorliegen. Gegebenenfalls können bei der Behandlung Dispergier-, Stabilisier-, Netz- und weitere Hilfsmittel zugesetzt werden.

Die neuen optischen Aufhellmittel gemäß vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgußmasse beifügen.

Sofern die Formgebung voll- oder halbsynthetischer organischer Materialien durch Spinnverfahren bzw. über Spinnmassen erfolgt, werden die optischen Aufheller vorzugsweise nach folgenden Verfahren appliziert:

- Zugabe zu den Ausgangssubstanzen (z.B. Monomeren) oder Zwischenprodukten (z.B. Vorkondensaten, Präpolymeren), d.h. vor oder während der Polymerisation, Polykondensation oder Polyaddition,
- Aufpudern auf Polymerisatschnitzel oder Granulate für Spinnmassen,
- Badfärbung von Polymerisatschnitzel oder Granulaten für Spinnmassen,
- Dosierte Zugabe zu Spinnschmelzen oder Spinnlösungen,
- Applikation auf Spinnkabel vor dem Verstrecken.

Eine bevorzugte Verwendung ist die Aufhellung von Polyacrylnitril in der Spinnmasse. Das so aufgehellte Polyacrylnitril hat den Vorteil, daß die Aufhellung auch beim Bleichen in Gegenwart von Aktivchlorspendern wie z.B. Hypochlorit, keine Verschlechterung des Effektes erleidet.

Die neuen optischen Aufhellmittel gemäß vorliegender Erfindung können beispielsweise auch in folgenden Anwendungsformen eingesetzt werden:

Mischungen mit Farbstoffen (Nuancierung) oder Pigmenten (Farb- oder insbesondere z.B. Weißpigmenten) oder als Zusatz zu Färbebädern, Druck-, Ätz- oder Reservepasten. Ferner auch zur Nachbehandlung von Färbungen, Drucken oder Ätzdrucken,

In Mischungen mit sogenannten "Carriern", Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),

In Mischung mit Vernetzern, Appareturmitteln (z.B. Stärke oder synthetischen Appreturen) sowie in Kombination mit den verschiedensten Textilveredlungsverfahren, insbesondere Kunstharzausrüstungen (z.B. Knitterfest-Ausrüstungen wie "wash-and-wear", "permanent-press", "non-iron"), ferner Flammfest-, Weichgriff-Schmutzablöse ("anti-soiling")- oder Antistatisch-Ausrüstungen oder antimikrobiellen Ausrüstungen,

Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Vliese, Papier, Leder,

In Kombination mit anderen, optisch aufhellend wirkenden Substanzen,

Als Scintillatoren, für verschiedene Zwecke photographischer Art, wie z.B. für elektrophotographische Reinproduktion oder Supersensibilisierung,

Je nach Substitution als Laser-Farbstoffe.

Die Menge der erfindungsgemäß zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind Mengen zwischen 0,005 und 2, vorzugsweise 0,1 bis 0,5 Gewichtsprozent von Interesse.

## B e i s p i e l e

### Beispiel 1

36 g (0,1 Mol) der Verbindung 22

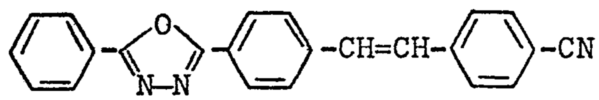 22

wurden in 500 ml Methylglykol mit 10 g Dicyandiamid und 2 g gepulvertem Kaliumhydroxyd 8 Stunden am Rückfluß gerührt. Anschließend wird bei Raumtemperatur abgesaugt, mit Methanol und anschließend mit Wasser neutral gewaschen und getrocknet. Man erhält 39 g (89 % d.Th.) der Verbindung

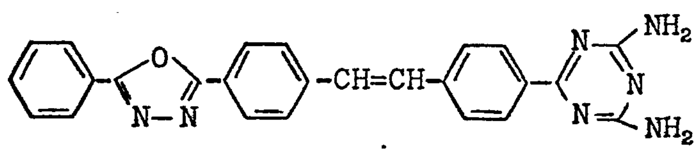 23

in Form eines hellgelben Pulvers mit einem Schmelzpunkt von 340 - 342 °C.

Analyse: $C_{25}H_{19}N_7O$ / 433,5 /

ber C 69,3; H 4,4; N 22,6
gef C 69,0; H 4,4; N 22,3

UV (gemessen in Dimethylformamid)

$\lambda$ max = 358 nm = 70 600

Die Ausgangsverbindung der Formel 22 ist beispielsweise auf folgenden Wegen zugänglich:

Beispiel 1 a

454,6 g (3 Mol) p-Cyanbenzylchlorid und 560,0 ml (3,3 Mol) Triäthylphosphit werden 5 Stunden auf 140 bis 145 °C erhitzt. Unter Abspaltung von Aethylchlorid entsteht nach einer Arbusow-Reaktion p-Cyan-benzyl-phosphonsäurediäthylester. Man kühlt das Reaktionsgemisch auf ca. 20°C ab, legt Hochvakuum an und erhitzt langsam auf ca. 100 °C Innentemperatur, um das überschüssige Triäthylphosphit abzudestillieren. Der rohe Phosphonsäureester kann ohne weitere Reinigung für die Folgereaktion eingesetzt werden.

Ausbeute: 751,5 g (99 %) Reinheit (GC): 96,1 %ig

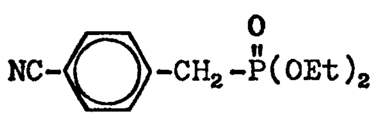

24

112,2 g KOH-Pulver (2 Mol) werden unter Stickstoff in 1 Liter DMF suspendiert. Unter Kühlung mit Eiswasser tropft man eine Lösung von 128 g Phosphonester (Verb. 24) und 75 g p-Carboxy-benzaldehyd (0,5 Mol) in 1 Liter DMF so zu, daβ die Innentemperatur 25 °C nicht übersteigt und rührt 12 Stunden bei Raumtemperatur nach. Das Reaktionsgemisch wird auf 5 Liter Eiswasser gegossen, das mit 200 ml konzentrierter Salzsäure angesäuert worden ist. Man erhitzt die Suspension kurz auf 90 °C, saugt den Kristallbrei nach dem Erkalten ab, wäscht mit Wasser neutral und trocknet. Man erhält 105,5 g (84,9 %) 4-Cyano-stilbencarbonsäure-(4') der Formel

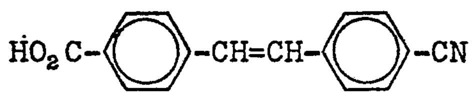 25

vom Schmp. 296 - 300 °C, welche nach Umkristallisieren aus Benzoesäuremethylester unter Klärung mit Tierkohle bei 301 - 302 °C schmilzt.

249 g ( 1 Mol ) 4-Cyano-stilbencarbonsäure-(4') Verb. 25 werden in 2500 ml Chlorbenzol mit 92 ml (0,11 Mol) Thionylchlorid 2 Stunden auf 70 - 100 °C erhitzt. Dann wird das überschüssige Thionylchlorid abdestilliert, auf Raumtemperatur abgekühlt, unter $N_2$-Atmosphäre abgesaugt, mit Chlorbenzol und Hexan gewaschen und getrocknet. Man erhält 242 g (90 % d.Th.) der Verbindung

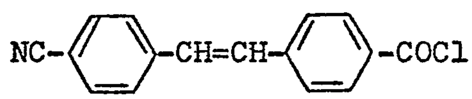 26

in Form eines gelben Pulvers vom Schmelzpunkt 188 - 190 °C, welches ohne Reinigung weiter umgesetzt wird.

70 g (0,5 Mol) Benzhydrazid werden in 1 000 ml Dioxan mit 55 ml Triäthylamin bei 10 °C vorgelegt und bei dieser Temperatur 135 g (0,5 Mol) der Verbindung 26 eingetragen. Anschließend 1 Stunde bei 10 °C und 1 Stunde bei Raumtemperatur gerührt, abgesaugt, mit Methanol und Wasser nachgewaschen und getrocknet. Man erhält 184 g ( 100 % d.Th.) der Verbindung,

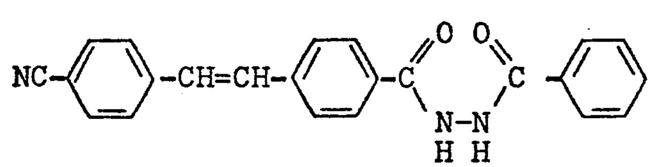 27

welche ohne weitere Reinigung in 1 800 ml o-Dichlorbenzol suspendiert wird. Zu dieser Suspension tropft man bei 80 °C 83 ml Thionylchlorid und rührt anschließend 8 Stunden am Rückfluß. Während dieser Zeit steigt die Innentemperatur auf 110 °C an. Nach dem Abkühlen auf Raumtemperatur wird abgesaugt, mit o-Dichlorbenzol und Methanol gewaschen und getrocknet. Man erhält 124 g (71 % d.Th.) der Verbindung 22 in Form eines hellgelben Pulvers vom Rohschmelzpunkt 248 - 250 °C, welches ohne weitere Reinigung umgesetzt werden kann.

Beispiel 1 b

6,3 g (20 mMol) der Verbindung der folgenden Formel

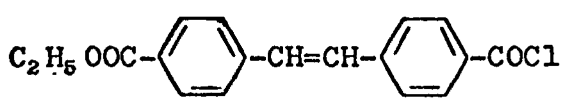 28

werden bei 0 °C in 50 ml Pyridin mit 2,8 g (20 mMol) Benzoesäurehydrazid versetzt und anschließend nach $1^h$ bei 0 °C, $1^h$ bei Raumtemperatur und schließlich $1^h$ bei Rückflußtemperatur gerührt. Die Reaktionsmischung wird nach Abkühlen auf 1 Liter Eis-Wasser gegossen, abgesaugt und frei von Chloridionen gewaschen. Nach dem Trocknen erhält man 7,0 g der Acylaminoverbindung.

Diese werden in 50 ml Thionylchlorid $24^h$ auf Rückflußtemperatur erwärmt. Danach ist in einer Probe keine Acylaminoverbindung dünnschichtchromatographisch mehr nachweisbar. Der Thionylchloridüberschuß wird abdestilliert, zuletzt unter Anlegen eines leichten Vakuums. Der Rückstand wird $1^h$ mit 25 ml Aethanol aufgekocht, abschließend kalt abgesaugt und mit Aethanol und Wasser frei von Chloridionen gewaschen. Man erhält 5,8 g der Verbindung der Formel

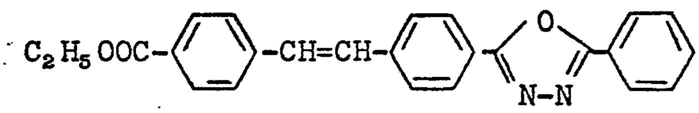

29

Zweimaliges Umkristallisieren aus Xylol unter Zuhilfenahme von Aktivkohle ergibt schwach gelbliche Kristalle vom Schmp. 241 °C. Ultraviolett-Absorptionsmaximum bei λ = 351 nm (gemessen in Dimethylformamid).

4,0 g der Verbindung 29 werden in 30 ml Dioxan mit 2 ml 10 N Natronlauge 5 Stdn. bei 85 - 90 °C gerührt, anschließend wird dieSuspension mit 10 ml Methanol verdünnt. Nach Abkühlen auf Raumtemperatur wird abgesaugt und mit Methanol gewaschen. Das feuchte Nutschgut wird in 100 ml Dimethylformamid eingetragen und bei 100 °C mit 5 ml konz. Salzsäure versetzt; nach Abkühlen wird abgesaugt, neutral gewaschen und getrocknet. Man erhält 3,0 g der Verbindung der Formel

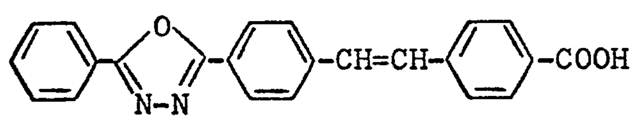

30

18,5 g (0,05 Mol) der Verbindung 30 werden in 200 ml Xylol mit 1 ml Dimethylformamid und 8 ml Thionylchlorid am Rückfluß gerührt bis eine klare Lösung vorhanden ist, anschließend wird der Ueberschuß Thionylchlorid abdestilliert, abgekühlt, abgesaugt, mit Xylol und Hexan gewaschen und getrocknet. Man erhält 17 g der Verbindung

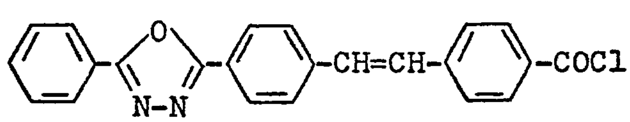

31

in Form eines gelben Pulvers vom Schmelzpunkt 232 - 240 °C welches ohne weitere Reinigung umgesetzt werden kann.

8 g (0,02 Mol) der Verbindung 31 werden unter $N_2$-Atmosphäre in 60 ml Dimethylformamid vorgelegt, 20 ml conz. Ammoniak zugegeben, 1 Stunde bei 25 °C, 1 Stunde bei 50 °C und 2 Stunden bei 100 °C gerührt, nach dem Abkühlen abgesaugt, mit Methanol und anschließend mit Wasser neutral gewaschen. Man erhält 7,3 g (99 % d.Th.) der Verbindung

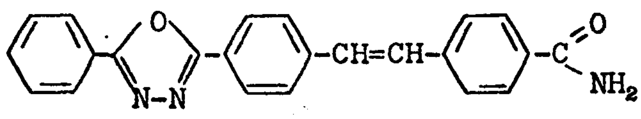

32

in Form eines beigen Pulvers vom Schmelzpunkt 271 - 275 °C, welches ohne weitere Reinigung in 70 ml o-Dichlorbenzol mit 3,5 ml Phosphoroxychlorid 24 Stunden am Rückfluß gerührt wird. Nach dem Abkühlen wird abgekühlt, abgesaugt, mit o-Dichlorbenzol, Methanol und Wasser neutral gewaschen und getrocknet. Man erhält 4,7 g (67,3 % d.Th.) der Verbindung 22.

Beispiel 1 c

Unter einer Stickstoffatmosphäre werden 2,3 g fein gepulvertes Kaliumhydroxid in 40 ml Dimethylformamid suspendiert. Unter leichter Kühlung wird eine Lösung von 2,6 g der Verbindung der Formel

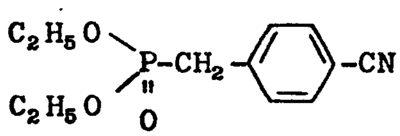

33

und 2,5 g der Verbindung der Formel

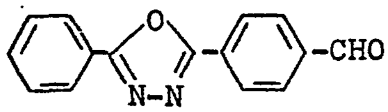

34

in 40 ml Dimethylformamid eingetropft. Die Temperatur des Reaktionsgemisches soll dabei 25 °C nicht übersteigen. Nach 20stündigem Rühren bei Raumtemperatur wird auf 100 ml Eis-Wasser, welches 4 ml konz. Salzsäure enthält, gegossen, kurz auf 90 °C erwärmt und nach dem Erkalten abgesaugt. Nach Neutralwaschen und Trocknen erhält man 2,6 g der Verbindung 22.

Die Verbindung der Formel 34 erhält man wie folgt:
23,6 g 2-Phenyl-5-(4-methylphenyl)-1,3,4-oxadiazol werden in 200 ml absolutem Tetrachlorkohlenstoff unter leichtem Erwärmen gelöst. Es werden 17.8 g N-Bromsuccinimid und 0,2 g Azoisobuttersäurenitril zugegeben und $4^h$ am Rückfluß gekocht. Es wird heiß abfiltriert, aus dem Filtrat kristallisieren beim Abkühlen 20,2 g der Verbindung der Formel

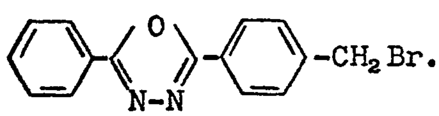

Aus Essigsäureäthylester erhält man farblose Kristalle vom Schmp. 170 - 172 °C.

15.7 g dieser Brommethylverbindung werden in 250 ml Chloroform gelöst und mit einer Lösung von 21 g Urotropin in 250 ml Chloroform vereinigt. Die Mischung wird $24^h$ bei Rückflußtemperatur gehalten, der ausgefallene Niederschlag wird kalt abgesaugt, mit Chloroform gewaschen und getrocknet. Der Rückstand wird in fein pulverisierter Form in einem Gemisch aus 135 ml Eisessig und 135 ml Wasser $2^h$ bei Rückflußtemperatur gehalten. Beim Abkühlen kristallisieren 7,2 (57 % d.Th.)

der Verbindung 34 aus. Aus Aethanol erhält man farblose Kristalle vom Schmp. 275-280 °C.

Analog Beispiel 1 a wurden folgende Verbindungen synthetisiert.

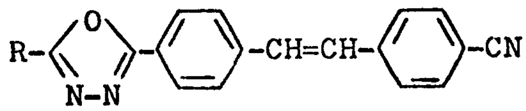 36

| Beispiel Nr. | R | Roh-Schmelzpunkt [°C] | Absorption [gem. in DMF] λmax [nm] |
|---|---|---|---|
| 2 a | 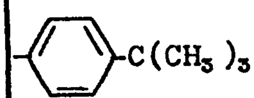 | 254 - 257 | 353 |
| 3 a | 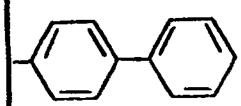 | 372 - 373 | 354 |
| 4 a | 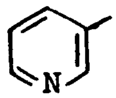 | 281 - 284 | 350 |
| 5 a | 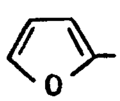 | 257 - 258 | 352 |
| 6 a | 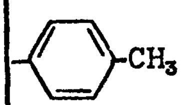 | 245 - 251 | 353 |
| 7 a | 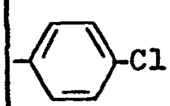 | 262 - 263 | 350 |
| 8 a | 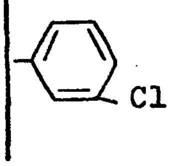 | 239 - 245 | 350 |

| Beispiel Nr. | R | Roh-Schmelzpunkt [°C] | Absorption [gem. in DMF] λmax [nm] |
|---|---|---|---|
| 9 a | 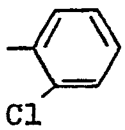 | 235 - 236 | 348 |
| 10 a | 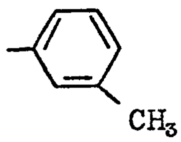 | 227 - 228 | 349 |
| 11 a | 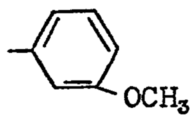 | 232 - 234 | 351 |
| 12 a | 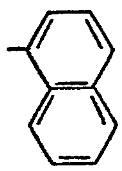 | 276 - 277 | 353 |
| 13 a | 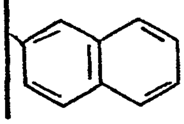 | 221 - 223 | 355 |

Analog Beispiel 1 wurden aus den Verbindungen 2 a - 13 a aus folgenden Verbindungen des Typs

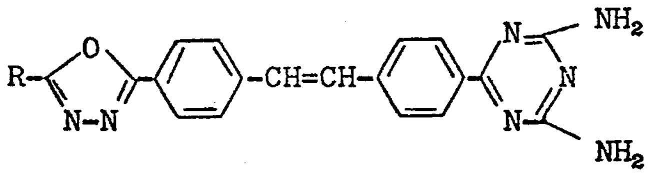

37

synthetisiert.

| Beispiel Nr. | R | Schmelzpunkt [°C] | Analyse N ber % | Analyse N gef % | Absorption [gem. in DMF] λ max [nm] |
|---|---|---|---|---|---|
| 2 | 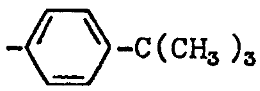 | 300 | 19,6 | 20,0 | 356 |
| 3 | 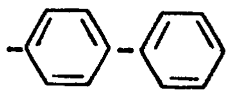 | 300 | 19,2 | 18,6 | 358 |
| 4 | 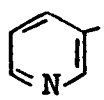 | 300 | 25,8 | 25,5 | 357 |
| 5 | 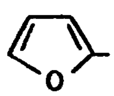 | 300 | 22,5 | 22,3 | 361 |
| 6 | 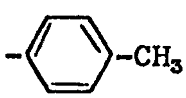 | 300 | 21,3 | 21,8 | 358 |
| 7 | 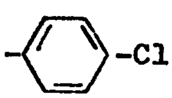 | 300 | 21,0 | 20,6 | 359 |
| 8 | 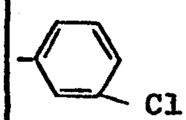 | 300 | 21,0 | 20,9 | 358 |
| 9 | 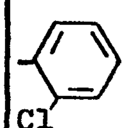 | 300 | 21,0 | 21,3 | 356 |
| 10 | 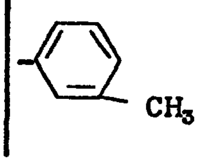 | 300 | 21,3 | 21,0 | |

| Beispiel Nr. | R | Schmelzpunkt [°C] | Analyse N ber % | gef % | Absorption [gem. in DMF] λ max [nm] |
|---|---|---|---|---|---|
| 11 | 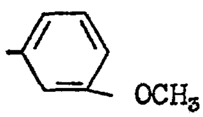 | 300 | 20,6 | 20,8 | 358 |
| 12 | 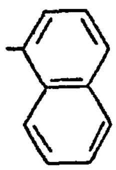 | 300 | 20,3 | 19,8 | 361 |
| 13 | 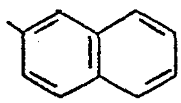 | 300 | 20,3 | 19,6 | 363 |

Beispiel 14

3,5 g (10 mMol) der Verbindung 38 werden in 50 ml Methylglykol

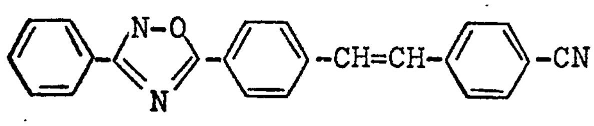 38

mit 1 g (12 Mol) Dicyandiamid und 0,3 g gepulvertem Kaliumhydroxyd 8 Stunden am Rückfluß gerührt. Anschließend wird bei Raumtemperatur abgesaugt, mit Methanol und anschließend mit Wasser neutral gewaschen und getrocknet. Man erhält 3,8 g (87,8 % d.Th.) der Verbindung

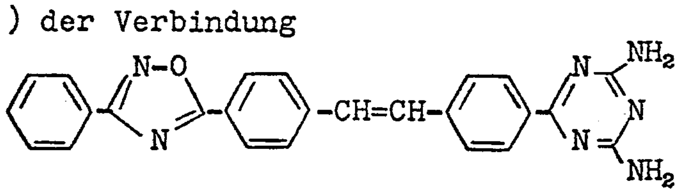 39

in Form eines hellgelben Pulvers, welches nach Umkristallisieren aus N-Methylpyrrolidon in Form farbloser Kristalle mit einem Schmelzpunkt > 300 °C anfällt.

Analyse: $C_{25}H_{19}N_7O$ /433,5/

| | | | |
|---|---|---|---|
| ber | C 69,3; | H 4,4; | N 22,6 |
| gef | C 68,9; | H 4,5; | N 22,6 |

Absorption (gemessen in Dimethylformamid):

$\lambda_{max}$ = 356 nm   $\varepsilon$ = 60 800

Beispiel 14 a

In eine Lösung von 8,2 g (60 mMol) Benzamidoxim in 150 ml Pyridin werden bei Raumtemperatur 15,7 g (50 mMol) der Verbindung der Formel

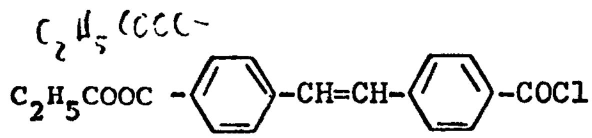

28

eingetragen, noch $1^h$ bei Raumtemperatur nachgerührt und anschließend $1^h$ am Rückfluß gekocht. Nach dem Abkühlen wird mit 1 Liter Eis-Wasser verdünnt, abgesaugt und frei von Chloridionen gewaschen. Nach dem Trocknen erhält man 13,6 g Acylaminoverbindung.

Diese werden in 100 ml DMF $4^h$ am Rückfluß gekocht. In einer Probe ist dann keine Acylaminoverbindung mehr dünnschichtchromatographisch nachweisbar. Aus der Reaktionsmischung fallen beim Abkühlen 7,5 g der Verbindung der Formel

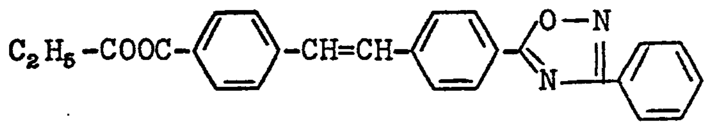 40

aus, die durch Umkristallisieren aus Xylol gerinigt werden. Man erhält fast farblose Kristalle vom Schmp. 235 °C, Ultraviolett-Absorptionsmaximum bei $\lambda$ = 345 nm (gemessen in Dimethylformamid).

4,0 g dieses Aethylesters werden in 30 ml Dioxan mit 2 ml 10 N Natronlauge 10 Stunden bei 75 °C gerührt, anschließend mit 10 ml Methanol verdünnt und nach Abkühlen auf Raumtemperatur abgesaugt. Das mit Methanol gewaschene, noch feuchte Nutschgut wird in 100 ml Dimethylformamid eingetragen und bei 100 °C mit 5 ml konz. Salzsäure versetzt. Es wird kalt abgesaugt, neutral gewaschen und getrocknet. Die Ausbeute an Carbonsäure der Formel

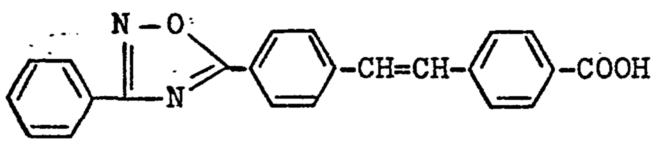 41

beträgt 2,9 g.

Diese Carbonsäure wird durch 5-stündiges Kochen im überschüssigen Thionylchlorid in das Säurechlorid der Formel

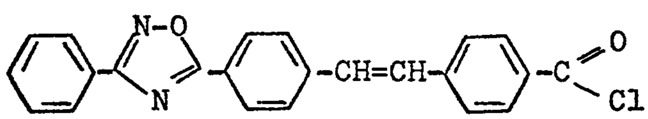 42

überführt. Nach Abdestillieren des Thionylchlorid-Ueberschußes, zuletzt mit Anlegen von Vakuum, besitzt das Säurechlorid den Schmelzpunkt von 212 °C.

3,9 g der Verbindung 42 werden unter Rühren und Eiskühlung in eine Mischung aus 20 ml Dimethylformamid und 20 ml konz. Ammoniak eingetragen, nach Abklingen der exothermen Reaktion noch $1^h$ bei Raumtemperatur, danach noch $1^h$ bei 95 - 100 °C gerührt. Das Produkt wird kalt abgesaugt und neutral gewaschen. Nach dem Trocknen erhält man 2,7 g Carbonsäureamid der Formel

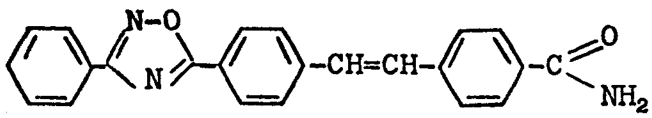

43

Diese werden in 25 ml Thionylchlorid $20^h$ am Rückfluß gekocht. Danach zeigt eine der Reaktionsmischung entnommene Probe im IR-Spektrum keine Carbonyl-Bande mehr. Der Thionylchlorid-Ueberschuß wird im Vakuum abdestilliert, der Rückstand wird in Eis-Wasser ausgerührt, abgesaugt und neutral gewaschen. Nach Trocknen erhält man 1,7 g Nitril der Formel 38. Umkristallisieren aus o-Dichlorbenzol unter Zuhilfenahme von Bleicherde liefert schwach gelbliche Kristalle vom Schmelzpunkt 219 - 221 °C.

Beispiel 14 b

3,4 g (25 mMol) Benzamidoxim werden in 50 ml Dimethylformamid mit 2 ml Triäthylamin vorgelegt und 5 g (20 mMol) der Verbindung 26 eingetragen. Anschließend wird 1 Stunde bei Raumtemperatur und 2 Stunden am Rückfluß gerührt, nach dem Abkühlen abgesaugt, mit Methanol und Wasser gewaschen und getrocknet. Man erhält 3,9 g (44,6 % d.Th.) der Verbindung 38 in Form farbloser Kristalle vom Schmelzpunkt 219 - 221 °C.

Für die Applikation auf Textilmaterial wurden je 100 mg der Verbindung aus Beispiel 1 (100 %-ig) in der Wärme in 10 ml Dimethylformamid gelöst und mit 5 ml eines Emulgators versetzt. Diese klare Lösung wurde unter Rühren in 85 ml einer wässrigen Lösung gegeben, die 100 ml/l des gleichen Emulgators enthält. Man erhielt so eine stabile Dispersion, die 1 g/l des optischen Aufhellers enthält.

Anwendungsbeispiel 1

Ein Gewebe aus Polyesterfasern wurde mit der oben beschriebenen Dispersion getränkt und zwischen zwei Gummiwalzen auf eine Restfeuchte von ca. 70 % abgequetscht. Dieses Gewebe wurde anschließend auf einem Spannrahmen einer Temperatur von 190 °C während 30 Sekunden ausgesetzt. Nach der dabei verlaufenden Trocknung und Fixierung wies das Gewebe einen sehr hohen Weißgrad auf, der deutlich höher als der eines nicht behandelten Gewebes war.

Anwendungsbeispiel 2

Von der oben beschriebenen Dispersion wurde eine solche Menge abpipettiert, die 0,1 % vom Warengewicht eines Polyamidgewebe entsprach. Diese Menge wurde in eine Flotte gegeben, die 1 g/l eines Bleichmittels enthielt. Nach einer Behandlung des Gewebes bei 98 °C während 1 Stunde in einem Flottenverhältnis von 1 : 15 wurde gespült und getrocknet. Das so behandelte Gewebe wies einen sehr guten Weißgrad auf, der deutlich über einem unbehandelten Gewebe lag.

Anwendungsbeispiel 3

Es wurde eine Lösung des Aufhellers aus Beispiel 1 b in Dimethylformamid hergestellt. Zu einer Spinnlösung aus Polyacrylnitril in Dimethylformamid wurde eine solche Menge der Aufhellerlösung zugegeben, daß, bezogen auf PAC, eine Menge von 200 ppm erreicht wurde. Die Spinnmasse wurde durch Spinndüsen gepreßt und das Lösungsmittel während des Abziehens der Fäden in der Trockenhitze verdampft. Nach dem Verstrecken der

Faser wies diese einen brillanten Weißton auf, der deutlich höher war, als der von Fäden, die keinen Aufheller enthielten.

Anwendungsbeispiel 4

Auf Granulat aus Polyester wurde eine solche Menge an Aufhellersubstrat in einem Mischer aufgepudert, daß, bezogen auf PES, 200 ppm erreicht wurden. Auf einer Spritzmaschine wurden aus diesem Granulat Spritzteile hergestellt. Bei einer Spritztemperatur von 260 °C wurden PES-Formteile erhalten, die einen ausgezeichneten Weißgrad von hoher Lichtechtheit aufwiesen.

Patentansprüche:

1. Diamino-1,3,5-triazinylstilben-Verbindungen der allgemeinen Formel

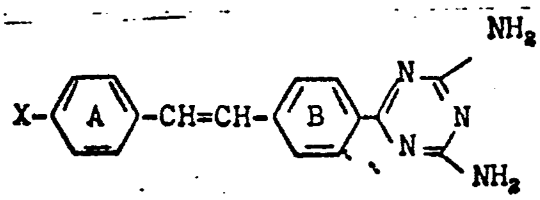

(I)

worin X eine Gruppe der Formeln

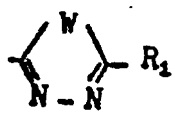

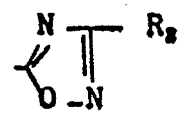

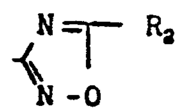

bedeutet und $R_1$ eine eventuell substituierte Aryl- oder Aralkenylgruppe oder eine heteroaromatische Gruppe ist, $R_2$ die gleiche Bedeutung wie $R_1$ hat und zusätzlich eine $(C_1-C_{18})$-Alkyl-, Cycloalkyl-, Aralkyl-, Alkoxyalkyl-, Halogenalkyl-, Hydroxyalkyl, Alkylaminoalkyl-, Carboxyalkylgruppe oder eine Gruppe der Formel $-(CH_2CH_2O)_n-R'$ mit R' = Wasserstoff oder $C_1-C_4$)-Alkyl und n = 1 oder 2 bedeuten, W ein Sauerstoff oder Schwefelatom bedeutet und die Ringe A und B noch weitere nicht-chromophore Substituenten aufweisen können.

2. Verbindungen nach Anspruch 1, wobei W ein Sauerstoffatom, $R_1$ einen Phenylring, der durch ein oder zwei Chloratome, eine oder zwei $(C_1-C_4)$-Alkyl- oder $(C_1-C_4)$-Alkoxy-$(C_1-C_4)$-alkyl-gruppen, eine Phenyl-, Cyano-, Carbonsäure-, Carbo-$(C_1-C_4)$-alkoxy-, Carbonsäureamid-, Sulfonsäure-, Sulfonsäureamid- oder Sulfonsäure-$(C_1-C_4)$-alkylestergruppe substituiert sein kann, $R_2$ die gleiche Bedeutung wie $R_1$ hat und zu-

sätzlich jeweils eine $(C_1-C_6)$-Alkyl-, $(C_1-C_6)$-Chloralkyl-, $(C_1-C_6)$-Alkoxy-$(C_1-C_4)$-alkyl-, $(C_1-C_4)$-Hydroxyalkyl-, Benzylgruppe oder eine Gruppe der Formel $-(CH_2CH_2O)_n-R'$ mit n = 1 oder 2 und R' = Wasserstoff oder $(C_1-C_4)$-Alkyl bedeutet.

3. Verbindungen nach Anspruch 1, wobei $R_1$ Phenyl, mono- oder di-$(C_1-C_2)$-Alkylphenyl, $(C_1-C_2)$-Alkoxyphenyl, Chlorphenyl oder Dichlorphenyl, $R_2$ die gleiche Bedeutung wie $R_1$ hat und zusätzlich jeweils $(C_1-C_5)$-Alkyl, $(C_1-C_2)$-Chloralkyl, $(C_1-C_2)$-Hydroxyalkyl, $(C_1-C_2)$-Alkoxyalkyl oder Benzyl, W Sauerstoff bedeutet und die Ringe A und B unsubstituiert sind.

4. Verbindungen nach Anspruch 1 der allgemeinen Formel

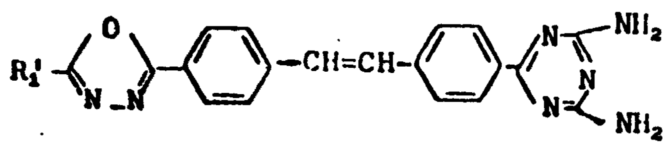

2

worin $R_1'$ Phenyl, mono oder di-$(C_1-C_2)$-Alkylphenyl, Chlorphenyl oder Dichlorphenyl bedeutet.

5. Verfahren zur Herstellung der Diamino-1,3,5-triazinylstilbenverbindungen nach Ansprüchen 1 bis 4, dadurch gekennzeichnet, daß man ein Nitril der Formel

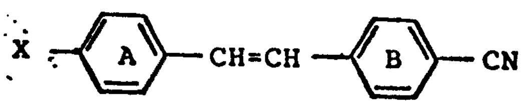

wobei X, A und B die in Ansprüchen 1 bis 4 genannten Bedeutungen haben, mit Dicyandiamid oder mit Harnstoff und Ammoniak umsetzt.

6. Verfahren zur Herstellung der Diamono-1,3,5-triazinylstilbenverbindungen nach Ansprüchen 1 bis 4, dadurch gekennzeichnet, daß man ein Amidinsalz der Formel

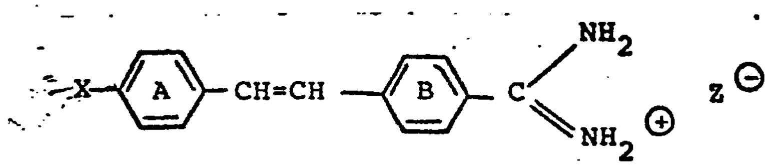

wobei X, A und B die in den Ansprüchen 1 bis 4 genannten Bedeutungen haben und Z ein Anion bedeutet, mit Dicyandiamid oder Biguanidsalzen umsetzt.

7. Verfahren zur Herstellung der Diamino-1,3,5-triazinyl-stilben-Verbindungen nach Ansprüchen 1 bis 3, dadurch gekennzeichnet, daß man ein Säurechlorid der Formel

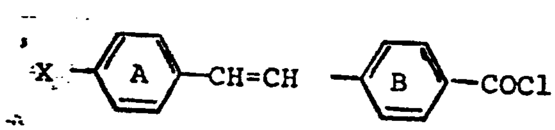

wobei X, A und B die in den Ansprüchen 1 bis 4 genannten Bedeutungen haben, mit einem Biguanidsalz umsetzt.

8. Verwendung der Diamino-1,3,5-triazinyl-stilben-Verbindungen nach Anspruch 1 bis 4 als optische Aufheller.

9. Verwendung der Diamino-1,3,5-triazinyl-stilben-Verbindungen nach Anspruch 1 bis 4 zum optischen Aufhellen von Polyacrylnitril in der Spinnmasse.

**Patentansprüche für Österreich:**

1. Verfahren zur Herstellung von Diamino-1,3,5-triazinyl-stilben-Verbindungen der allgemeinen Formel 1

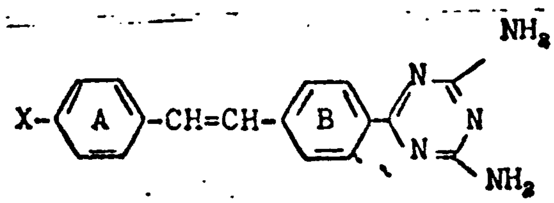

(1)

worin X eine Gruppe der Formeln

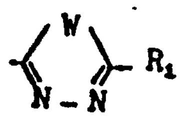

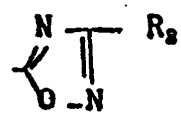

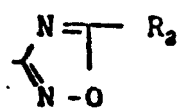

bedeutet und $R_1$ eine eventuell substituierte Aryl- oder Aralkenylgruppe oder eine heteroaromatische Gruppe ist, $R_2$ die gleiche Bedeutung wie $R_1$ hat und zusätzlich eine $(C_1-C_{18})$-Alkyl-, Cycloalkyl-, Aralkyl-, Alkoxyalkyl-, Halogenalkyl-, Hydroxyalkyl, Alkylaminoalkyl-, Carboxyalkylgruppe oder eine Gruppe der Formel $-(CH_2CH_2O)_n-R'$ mit R' = Wasserstoff oder $C_1-C_4$)-Alkyl und n = 1 oder 2 bedeuten, W ein Sauerstoff oder Schwefelatom bedeutet und die Ringe A und B noch weitere nicht-chromophore Substituenten aufweisen können, **dadurch gekennzeichnet, daß man Carbonsäuren der allgemeinen Formel 3**

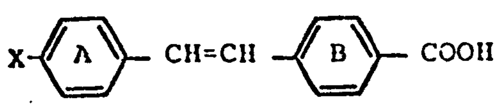

3

**in der X, A und B die obengenannten Bedeutungen haben, oder deren Chloride, Nitrile oder Amidinsalze durch Umsetzung mit Dicyandiamid, Biguanidsalzen bzw. Harnstoff und Ammoniak in die entsprechende Diamino-1,3,5-**

0008123



triazinyl-Verbindungen überführt.

2. Verfahren zur Herstellung von Diamino-1,3,5-triazinyl-stilben-Verbindungen nach Anspruch 1, wobei W ein Sauerstoffatom, $R_1$ einen Phenylring, der durch ein oder zwei Chloratome, eine oder zwei $(C_1-C_4)$-Alkyl- oder $(C_1-C_4)$-Alkoxy-$(C_1-C_4)$-alkyl-gruppen, eine Phenyl-, Cyano-, Carbonsäure-, Carbo-$(C_1-C_4)$-alkoxy-, Carbonsäureamid-, Sulfonsäure-, Sulfonsäureamid- oder Sulfonsäure-$(C_1-C_4$-alkylestergruppe substituiert sein kann, $R_2$ die gleiche Bedeutung wie $R_1$ hat und zusätzlich jeweils eine $(C_1-C_6)$-Alkyl-, $(C_1-C_6)$-Chloralkyl-, $(C_1-C_6)$-Alkoxy-$(C_1-C_4)$-alkyl-, $(C_1-C_4)$-Hydroxyalkyl-, Benzylgruppe oder eine Gruppe der Formel $-(CH_2CH_2O)_n-R'$ mit n = 1 oder 2 und R' = Wasserstoff oder $(C_1-C_4)$-Alkyl bedeutet.

3. Verfahren zur Herstellung von Diamino-1,3,5-triazinyl-stilben-Verbindungen nach Anspruch 1, wobei $R_1$ Phenyl, mono- oder di-$(C_1-C_2)$-Alkylphenyl, $(C_1-C_2)$-Alkoxyphenyl, Chlorphenyl oder Dichlorphenyl, $R_2$ die gleiche Bedeutung wie $R_1$ hat und zusätzlich jeweils $(C_1-C_5)$-Alkyl, $(C_1-C_2)$-Chloralkyl, $(C_1-C_2)$-Hydroxyalkyl, $(C_1-C_2)$-Alkoxyalkyl oder Benzyl, W Sauerstoff bedeutet und die Ringe A und B unsubstituiert sind.

4. Verfahren zur Herstellung von Verbindungen der allgemeinen Formel 2

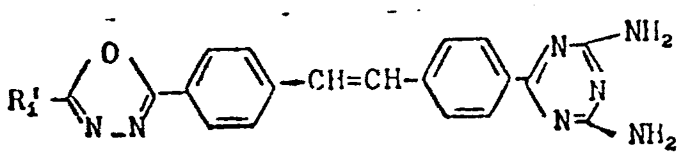

(2)

worin $R_1$ Phenyl, mono oder di-$(C_1-C_2$-Alkylphenyl, Chlorphenyl oder Dichlorphenyl bedeutet, nach der Verfahrensweise gemäß Anpspruch 1.

5. Verfahren zur Herstellung der Diamino-1,3,5-triazinyl-stilben-Verbindungen nach Ansprüchen 1 bis 4, dadurch gekennzeichnet, daß man ein Nitril der Formel

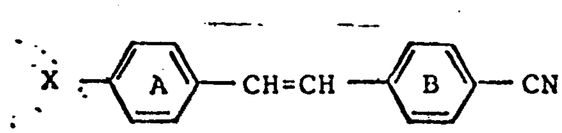

wobei X, A und B die in Ansprüchen 1 bis 4 genannten Bedeutungen haben, mit Dicyandiamid in An- oder Abwesenheit basischer Katalysatoren umsetzt.

6. Verwendung von Diamino-1,3,5-triazinylstilben-Verbindungen der allgemeinen Formel 1

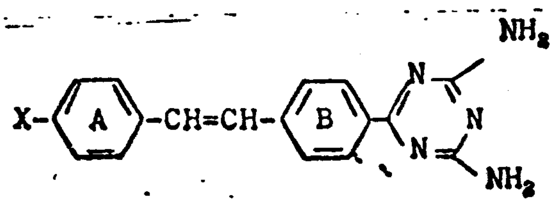

(1)

worin X eine Gruppe der Formeln

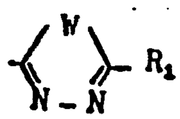

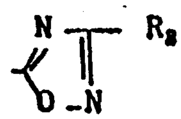

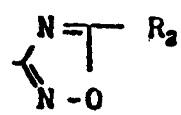

bedeutet und $R_1$ eine eventuell substituierte Aryl- oder Aralkenylgruppe oder eine heteroaromatische Gruppe ist, $R_2$ die gleiche Bedeutung wie $R_1$ hat und zusätzlich eine $(C_1$-$C_{18})$-Alkyl-, Cycloalkyl-, Aralkyl-, Alkoxyalkyl-, Halogenalkyl-, Hydroxyalkyl, Alkylaminoalkyl-, Carboxyalkylgruppe oder eine Gruppe der Formel $-(CH_2CH_2O)_n-R'$ mit R' = Wasserstoff oder $C_1$-$C_4$)-Alkyl und n = 1 oder 2 bedeuten, W ein Sauerstoff oder Schwefelatom bedeutet und die Ringe A und B noch weitere nicht-chromophore Substituenten aufweisen können, als optische Aufheller.

7. Verwendung der Diamino-1,3,5-triazinylstilben-Verbindungen der allgemeinen Formel 1

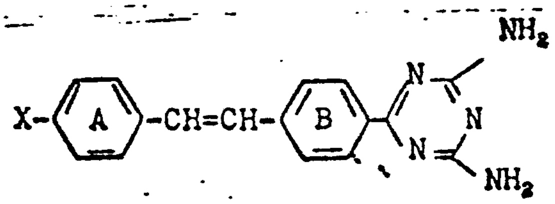

(1)

worin X eine Gruppe der Formeln

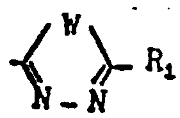

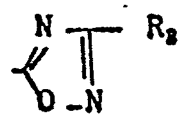

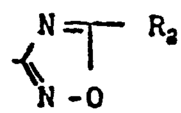

bedeutet und $R_1$ eine eventuell substituierte Aryl- oder Aralkenylgruppe oder eine heteroaromatische Gruppe ist, $R_2$ die gleiche Bedeutung wie $R_1$ hat und zusätzlich eine $(C_1-C_{18})$-Alkyl-, Cycloalkyl-, Aralkyl-, Alkoxyalkyl-, Halogenalkyl-, Hydroxyalkyl, Alkylaminoalkyl-, Carboxyalkylgruppe oder eine Gruppe der Formel $-(CH_2CH_2O)_n-R'$ mit R' = Wasserstoff oder $C_1-C_4$)-Alkyl und n = 1 oder 2 bedeuten, W ein Sauerstoff oder Schwefelatom bedeutet und die Ringe A und B noch weitere nicht-chromophore Substituenten aufweisen können, zum optischen Aufhellen von Polyacrylnitril in der Spinnmasse.

8. Verwendung der nach den Verfahren gemäß Ansprüche 2 bis 5 erhältlichen Diamino-1,3,5-triazinylstilben-Verbindungen als optische Aufheller.

9. Verwendung der nach den Verfahren gemäß Ansprüche 2 bis 5 erhältlichen Diamino-1,3,5-triazinylstilben-Verbindungen zum optischen Aufhellen von Polyacrylnitril in der Spinnmasse.

Europäisches Patentamt

# EUROPÄISCHER RECHERCHENBERICHT

Nummer der Anmeldung
EP 79 10 2893

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| E | FR - A - 2 384 827 (BAYER)<br>* Ansprüche 1,2; Seite 7, Zeilen 23,24; Seite 10, Zeile 29 *<br>-- | 1-4,8,9 |
| A | DE - A - 1 919 209 (HOECHST)<br>* Anspruch 1 *<br>---- | 1 |

KLASSIFIKATION DER ANMELDUNG (Int.Cl. 3)

C 07 D 413/10
417/10
D 06 L 3/12
C 08 K 5/35
5/47
C 08 J 3/20

RECHERCHIERTE SACHGEBIETE (Int. Cl. 3)

C 07 D 413/10
417/10
D 06 L 3/12

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 22-10-1979 | GINESTET |